(19) Europäisches Patentamt
European Patent Office
Office européen des brevets

(11) **EP 2 208 462 A1**

(12) **EUROPEAN PATENT APPLICATION**
published in accordance with Art. 153(4) EPC

(43) Date of publication:
**21.07.2010 Bulletin 2010/29**

(51) Int Cl.:
**A61B 5/055** (2006.01)

(21) Application number: **08833547.6**

(22) Date of filing: **25.09.2008**

(86) International application number:
**PCT/JP2008/067344**

(87) International publication number:
**WO 2009/041534 (02.04.2009 Gazette 2009/14)**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **25.09.2007 JP 2007246841**

(71) Applicant: **Gifu University**
**Gifu-shi**
**Gifu 501-1193 (JP)**

(72) Inventors:
• **KINOSADA, Yasutomi**
**Gifu-shi**
**Gifu 501-1193 (JP)**

• **MATSUSHIMA, Shigeru**
**Gifu-shi**
**Gifu 501-1193 (JP)**
• **ERA, Seiichi**
**Gifu-shi**
**Gifu 501-1193 (JP)**
• **OSADA, Shinji**
**Gifu-shi**
**Gifu 501-1193 (JP)**

(74) Representative: **Jackson, Martin Peter**
**J.A. Kemp & Co.**
**14 South Square**
**Gray's Inn**
**London WC1R 5JJ (GB)**

(54) **MAGNETIC RESONANCE IMAGING APPARATUS AND ITS OPERATING METHOD, DIAGNOSTIC IMAGING SYSTEM, AND DIAGNOSIS METHOD**

(57)     The present teaching aims to detect a lesion in tissue at a very early stage. The present teaching provides a magnetic resonance imaging apparatus 10 including a transmission/reception module 26 that receives a magnetic resonance signal from a subject after transmitting a high-frequency signal, a control module 40 that controls the transmission/reception module such that scans are executed based on pulse sequences corresponding to a first imaging condition without application of an MT pulse and to a second imaging condition accompanied by the application of the MT pulse, a calculation module 36 that calculates a lesion diagnostic rating index represented as a function of a signal intensity Mo of a first magnetic resonance signal received from the subject according to the first imaging condition and a signal intensity Ms of a second magnetic resonance signal received from the subject according to the second imaging condition, and a detection module 38 that detects a lesion in living tissue or a presage thereof in the imaged region based on the lesion diagnostic rating index.

Fig.5

**Description**

Technical Field

The present teaching relates to a magnetic resonance imaging device and an operating method therefor, a diagnostic imaging system, and a diagnosis method.

Background Art

Early detection and early treatment of lesions is strongly required in the field of health care not only for the sake of patients but also from the viewpoint of health care costs. For example, diagnostic imaging by magnetic resonance imaging (MRI) is a useful technique for discovering tissue lesions and abnormalities. A technique that applies magnetization transfer (MT) effect has been proposed as an imaging technology that uses MRI (cf. e.g., Patent Documents 1 and 2).

In addition, the inventors of the present teaching previously proposed a new indicator in the form of equivalent cross-relaxation rate (ECR) that constructs images by emphasizing portions affected by MT effects (cf. e.g., Non-Patent Document 1). This Non-Patent Document 1 discloses that, in the case of comparing the ECR of breast cancer metastatic lymph node tissue and normal tissue, the breast cancer metastatic lymph node tissue exhibits lower values than normal tissue.

    Patent Document 1: Japanese Patent Application Laid-open No. H11-313810
    Patent Document 2: Japanese Patent Application Laid-open No. 2006-116299
    Non-Patent Document 1: Magnetic Resonance in Medicine, 2005, 54, pp. 1300-1304

Disclosure of the Invention

In general, tissue lesions are only subjected to testing and detected after a functional abnormality of a tissue appears in the form of an abnormal value in a blood test or biochemical test and the like. However, there are cases in which the tissue lesion may have already progressed when biochemical test data or other data has indicated an abnormal value. In addition, since conventional MR diagnostic imaging technology determines a disease state based on morphological changes in tissue or changes in blood flow and the like, lesions were unable to be discovered until abnormalities in tissue function appeared. Moreover, with respect to the ECR previously proposed by the inventors of the present teaching, although evaluations are carried out with respect to the properties of body tissue in the manner of a difference in ECR between cancer tissue and normal tissue, the relationship between the degree of progression of a lesion and ECR was unclear.

In this manner, diagnosis of a disease has conventionally consisted primarily of a diagnosis of the presence of abnormal test values or tissue morphological abnormalities, and did not involve detection of a presage (predictive information) of a tissue lesion in the form of an abnormality. Thus, under the present circumstances, it was not possible to realize early treatment by detecting even a slight tissue lesion as the presage of a disease at the stage at which functional abnormalities or changes in tissue morphology had not yet appeared.

With this in mind, an object of the present teaching is to provide a magnetic resonance imaging apparatus capable of extremely early detection of tissue lesions, an operating method thereof, a diagnostic imaging system and a diagnosis method.

The inventors of the present teaching made a comparison of patient blood and/or biochemical test values with a tissue diagnostic rating index, which is represented as a function of a signal intensity Mo and a signal intensity Ms of a second magnetic resonance signal received from a subject according to a second imaging condition, to examine whether or not the lesion diagnostic rating index is able to reflect subtle changes in the molecular structure of a tissue to be diagnosed. Whereupon, the lesion diagnostic rating index was surprisingly found to allow sensitive detection and diagnosis of presages or the presence of tissue abnormalities at an earlier stage than the appearance of abnormalities in blood and biochemical test values. Namely, it was found that the lesion diagnostic rating index had already demonstrated values capable of being distinguished from those of healthy subjects at the stage at which MRI and biochemical data had not yet reached an abnormal range. Moreover, it was also found that biochemical test values subsequently reach an abnormal range in patients distinguished from health subjects on the basis of the lesion diagnostic rating index. The inventors of the present teaching completed the present teaching on the basis of these findings. Namely, the followings are provided by the present teaching.

According to the present teaching, a magnetic resonance imaging apparatus is provided that includes: a transmission/reception module configured to receive a magnetic resonance signal from a subject after transmitting a high-frequency signal; a control module configured to control the transmission/reception module such that scans are executed based on pulse sequences corresponding to a first imaging condition unaccompanied by application of an MT pulse and to a second imaging condition accompanied by application of the MT pulse; a calculation module configured to calculate a lesion diagnostic rating index represented as a function of a signal intensity Mo of a first magnetic resonance signal received from the subject according to the first imaging condition and a signal intensity Ms of a second magnetic resonance signal received from the subject according to the second imaging condition; and a detection module configured to detect a lesion in living tissue or a presage thereof in the imaged region based on the lesion diagnostic rating index. The lesion diagnostic rating index may be calculated based on the following equation. The lesion diagnostic rating index represented by the following equation (1) is the ECR previously proposed by the inventors of the present teaching.

(Equation 1)

$$ECR(\%) = ((Mo\text{-}Ms)/Ms) \times 100 \tag{1}$$

According to the present teaching, the apparatus may also be provided in which the detection module is a module configured to detect the lesion in living tissue or the presage thereof based on time-based changes in the lesion diagnostic rating index. Further, the detection module may be a module configured to detect the lesion in living tissue or the presage thereof based on threshold information on the lesion diagnostic rating index that has been correlated with testing information relating to a patient for which a definitive diagnosis has been made according to a method differing from diagnosis according to the lesion diagnostic rating index.

According to the present teaching, the detection module may be a module configured to perform detection based on the threshold information on the lesion diagnostic rating index that has been correlated with testing information relating to blood of the patient.

The detection module may further be a module configured to detect the lesion in living tissue or the presage thereof based on the threshold information. In this aspect, the detection module may be a module configured to detect normalcy of a tissue when a value of the tissue diagnostic rating index exceeds a prescribed threshold information E1, detect a presage of a tissue lesion when the value of the tissue diagnostic rating index has exceeded a prescribed threshold information E2 but is equal to or less than the prescribed threshold information E1, and detect a presence of the tissue lesion when the value of the tissue diagnostic rating index is equal to or less than the prescribed threshold information E2.

Moreover, according to the present teaching, the apparatus may also be provided in which the detection module is a module configured to perform detection based on a use of a correlation between testing information other than tissue diagnostic rating index obtained from a healthy subject group and a patient group, for which a definitive diagnosis has been made according to a method differing from diagnosis using the tissue diagnostic rating index and the lesion diagnostic rating index that has been correlated with this testing information, and also based on a lesion diagnostic rating index of a subject and the subject's testing information other than his/her lesion diagnostic rating index.

According to the present teaching, the living tissue may be liver tissue, and the testing information may be hematological information on one type or two or more types of components selected from a group consisting of glutamic oxaloacetic transaminase (GOT), glutamic pyruvic transaminase (GPT), albumin, bilirubin, cholinesterase and indocyanine green (ICG).

In addition, the MT pulse in the present teaching may be that in which an irradiation frequency is near a resonance frequency of water.

According to the present teaching, an operating method for a magnetic resonance imaging apparatus may be provided that comprises: a calculation step of calculating a lesion diagnostic rating index represented as a function of a first magnetic resonance signal Mo received from a subject according to a first imaging condition unaccompanied by application of an MT pulse and a second magnetic resonance signal Ms received from the subject according to a second imaging condition accompanied by application of the MT pulse, and a detection step of detecting a presage of a lesion in living tissue in an imaged region based on the lesion diagnostic rating index.

According to the present teaching, a diagnostic imaging system may be provided that is provided with a transmission/reception module configured to receive a magnetic resonance signal from a subject after transmitting a high-frequency signal; a control module configured to control the transmission/reception module such that scans are executed based on pulse sequences corresponding to a first imaging condition unaccompanied by application of an MT pulse and to a second imaging condition accompanied by application of the MT pulse; a calculation module configured to calculate a lesion diagnostic rating index represented as a function of a signal intensity Mo of a first magnetic resonance signal received from the subject according to the first imaging condition and a signal intensity Ms of a second magnetic resonance signal received from the subject according to the second imaging condition; and a detection module configured to detect a presage of a lesion in living tissue in the imaged region based on the lesion diagnostic rating index.

According to the present teaching, a method of detecting a lesion in living tissue may be provided that comprises: a detection step of detecting a lesion in living tissue or a presage thereof based on a lesion diagnostic rating index represented as a function of a signal intensity Mo of a first magnetic resonance signal received from a subject according to a first imaging condition unaccompanied by application of an MT pulse and a signal intensity Ms of a second magnetic resonance signal received from the subject according to a second imaging condition accompanied by application of the MT pulse.

According to the present teaching, a tissue lesion detection apparatus may be provided that is provided with a module configured to acquire a lesion diagnostic rating index represented as a function of a signal intensity Mo of a first magnetic resonance signal received from an imaged region of a subject according to a first imaging condition unaccompanied by application of an MT pulse and a signal intensity Ms of a second magnetic resonance signal received from the imaged region of the subject according to a second imaging condition accompanied by application of the MT pulse, and a detection module configured to detect a lesion in living tissue or a presage thereof in the imaged region based on the lesion diagnostic rating index.

The detection apparatus of the present teaching may further be provided with an imaging module configured to image a degree of the lesion in the imaged region based on the lesion diagnostic rating index in the imaged region, and an output module configured to output an image of the degree of the lesion based on the lesion diagnostic rating index.

Brief Description of the Drawings

FIG. 1 is an explanatory drawing showing an example of a pulse train used in imaging for calculating a lesion diagnostic rating index.

FIG. 2 is an explanatory drawing showing an example of a graph for setting threshold information of a lesion diagnostic rating index.

FIG. 3 is an explanatory drawing showing an example of threshold information of a lesion diagnostic rating index.

FIG. 4 is an explanatory drawing showing an example of using a lesion diagnostic rating index and testing information relating to blood to detect a lesion.

FIG. 5 is a block diagram showing the general configuration of a magnetic resonance imaging apparatus 10 of the present teaching.

FIG. 6 is a flow chart illustrating an operating method for a magnetic resonance imaging apparatus 10 of the present teaching.

FIG 7 is a drawing illustrating correlations between lesion diagnostic rating index data and blood data of Example 1, wherein FIG. 7(a) represents the correlation between lesion diagnostic rating index data and GOP, FIG. 7(b) between that and GPT, FIG. 7(c) between that and albumin, FIG. 7(d) between that and bilirubin, FIG. 7(e) between that and cholinesterase, and FIG. 7(f) between that and ICG.

FIG. 8 is a drawing indicating time-based changes in blood data following calculation of lesion diagnostic rating index of Example 2, wherein FIG. 8(a) indicates the changes in albumin, and FIG. 8(b) indicates the changes in cholinesterase.

FIG. 9 is a graph indicating cell densities and lesion diagnostic rating indices for normal tissue and abnormal tissue.

FIG. 10 is a drawing showing the results of classifying a cirrhosis group, a hepatitis group and a normal group based on combinations of blood test results.

FIG. 11 is a drawing showing a graph of investigating a distribution of lesion diagnostic rating indices among patients included in each of the groups shown in FIG. 10.

FIG. 12 is a drawing showing a graph of the numbers of patients of each of the groups shown in FIG. 10 contained with the range of each lesion diagnostic rating index.

FIG. 13 is a drawing showing time-based changes (1 month) in lesion diagnostic rating indices and platelet test results in 11 subjects.

Best Mode for Carrying Out the Invention

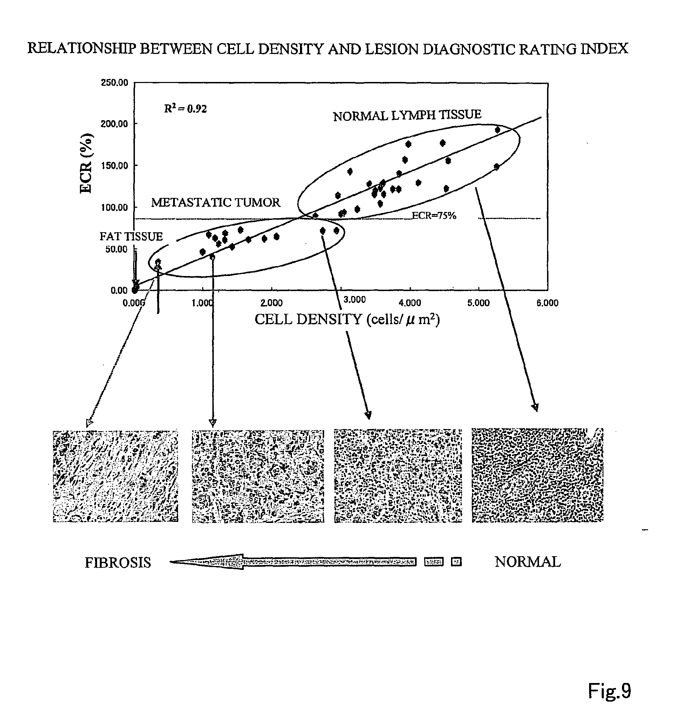

According to an MRI apparatus, diagnosis method and the like of the present teaching, minute structural changes in tissue molecules can be accurately determined by using a lesion diagnostic rating index as an indicator of a presage or presence of a tissue lesion. The lesion diagnostic rating index is represented as a function of a signal intensity Mo and a signal intensity Ms, where the signal intensity Mo is of a first magnetic resonance signal received from an imaged region of a subject according to a first imaging condition unaccompanied by application of an MT pulse, and the signal intensity Ms is of a second magnetic resonance signal received from the imaged region of the subject according to a second imaging condition accompanied by the application of the MT pulse. According to the inventors of the present teaching, the lesion diagnostic rating index is known to demonstrate values in tumor tissue or other diseased tissue that differ from those of normal tissue. However, as shown in FIG. 9, when cell density decreases accompanying tissue fibrosis due to tumor metastasis and the like from normal tissue, the lesion diagnostic rating index also decreases, and when cell density is plotted versus lesion diagnostic rating index for normal tissue and abnormal tissue (referring here to tumor metastatic tissue), both tissues were determined to be able to be clearly distinguished on the basis of lesion diagnostic rating indices. In addition, it was determined for the first time here that the lesion diagnostic rating index demonstrates abnormal values that can already be distinguished from healthy subjects even at a stage prior to detection of an abnormality on the basis of diagnostic imaging or biochemical testing data, namely at a stage at which abnormalities would be overlooked as being 'normal' by the conventional techniques. Moreover, the lesion diagnostic rating index was also determined to decrease starting at the stage of presages to the stage at which tissue lesions are detected even by using other diagnostic techniques and corresponding to the degree of subsequent progression thereof.

Although the new finding obtained here that the lesion diagnostic rating index can serve as an indicator for diagnosing the presence of a tissue lesion and indicator for detecting a tissue lesion at the stage of a presage (indicator for diagnosing a presage) is thought to be based on the lesion diagnostic rating index being able to detect a change in tissue microstructure, there had been no correlation made whatsoever between the detection performance thereof and tissue lesion presages. In the past, the lesion diagnostic rating index was only used to reflect subtle changes in the state of diseased tissue.

On the basis of the above, the present teaching is able to provide a novel diagnosis method and apparatus capable of early detection of tissue lesions. Moreover, since the present teaching enables detection of tissue lesions from the stage of a presage to an advanced stage both non-invasively and quantitatively, it is extremely useful as a method for performing regular health examinations on healthy subjects as well as a first diagnostic method of choice at the stages of diagnosis and treatment.

In addition, since the present teaching can be carried out simply by adding a function enabling detection of MT effects to an ordinary MRI apparatus used in the routine clinical setting, the present teaching does not require any special devices and has a high degree of universality. Moreover, since the present teaching only requires processing of image data captured with an MRI apparatus, lesions can be detected both quickly and easily. In this manner, the lesion diagnostic rating index is extremely useful as a novel indicator for early discovery and diagnosis of tissue lesions.

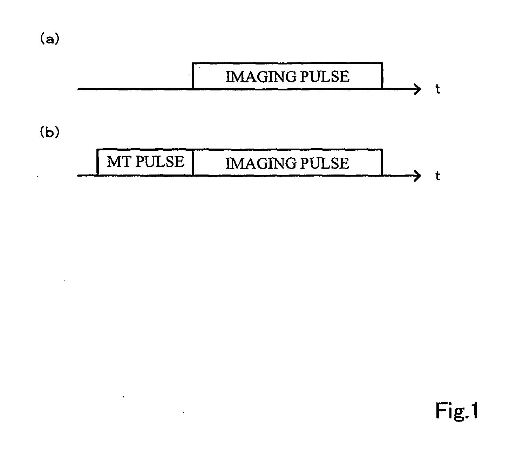

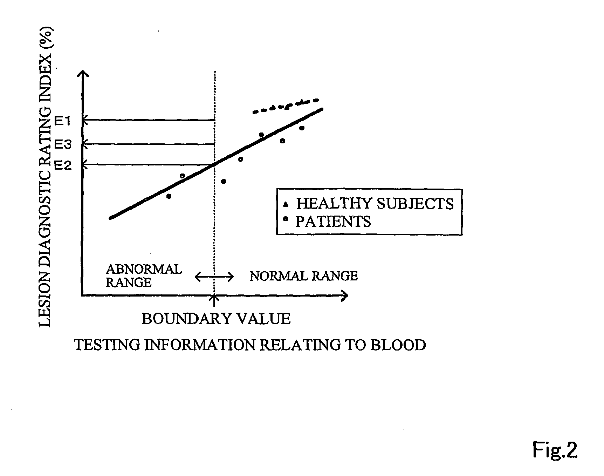

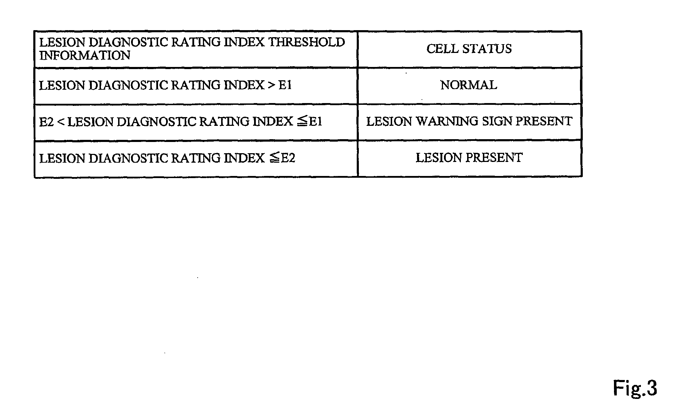

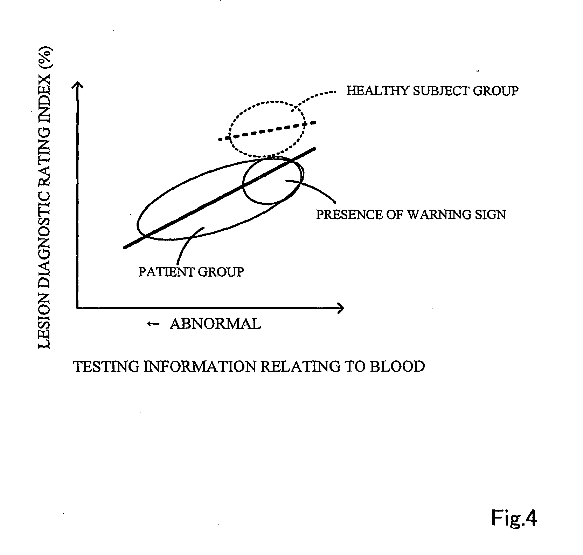

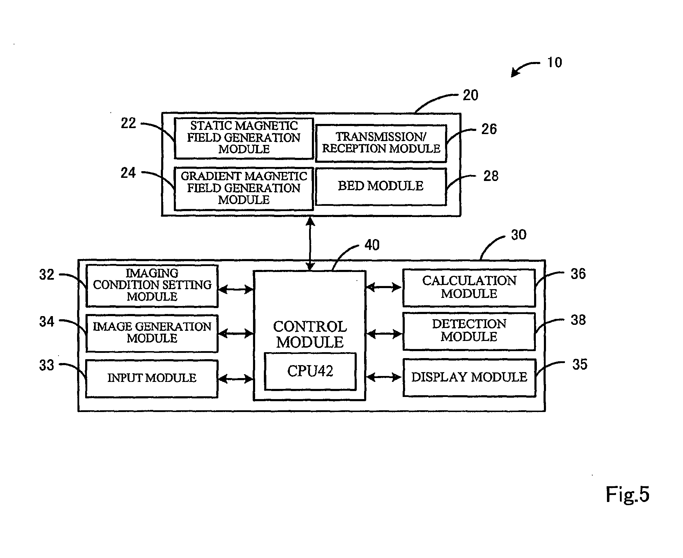

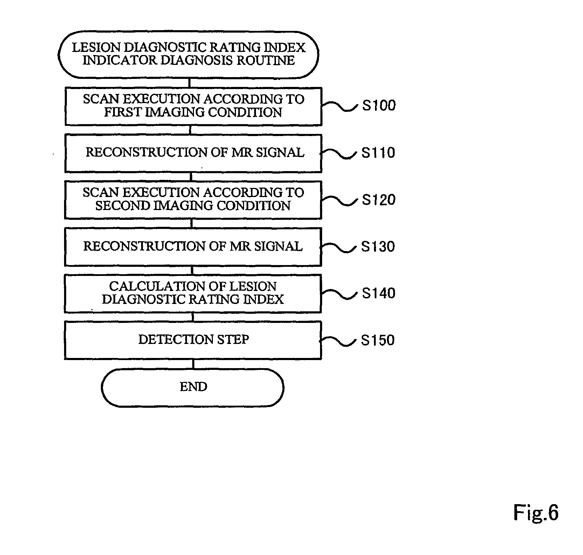

In addition to a magnetic resonance imaging apparatus, the present teaching also provides an operating method thereof, a diagnostic imaging system, and a diagnosis method. The following provides a detailed explanation of embodiments of the present teaching while suitably using the drawings. Here, FIG. 1 is an explanatory drawing showing an example of a pulse train used during imaging for calculating a lesion diagnostic rating index, FIG. 2 is an explanatory drawing showing an example of a graph for setting threshold information of a lesion diagnostic rating index, FIG. 3 is an explanatory drawing showing an example of threshold information of a lesion diagnostic rating index, FIG. 4 is an explanatory drawing showing an example of the use of a lesion diagnostic rating index and testing information relating to blood to detect a lesion, FIG. 5 is a block diagram showing the general configuration of a magnetic resonance imaging apparatus 10 of the present teaching, and FIG. 6 is a flow chart illustrating an operating method for a magnetic resonance imaging apparatus 10. Furthermore, although the embodiments indicated below are preferred embodiments of the present teaching; they are not intended to limit the present teaching.

(Diagnosis Method)

The diagnosis method of the present teaching is a method for diagnosing a lesion in living tissue by analyzing information relating to a lesion diagnostic rating index obtained from a subject to be diagnosed. There are no particular limitations on the subject of the present teaching, and the subject may be a human or non-human animal. Examples of non-human animals include livestock, poultry and pets. In addition, there are also no particular limitations on the type of body tissue, and tissue of an organ of the head region, thoracic region, abdominal region, lumbar region or any other region throughout the entire body can be used as a subject tissue. Preferably, the organ is an organ that allows the progression of a lesion to be correlated with factors that increase or decrease in the body and more preferably testing information in which the factors are related to the blood, and even more preferably the organ is the liver.

There are also no particular limitations on the type of lesion to be diagnosed. For example, in the case of the liver, the diagnosis method can be applied to diagnosis of various types of hepatitis such as acute hepatitis, chronic hepatitis or alcoholic hepatitis, as well as various liver function disorders such as cirrhosis or hepatocellular carcinoma.

(Detection Step)

The diagnosis method is provided with a detection step. The detection step is a step of detecting the lesion in living tissue or presage thereof based on the lesion diagnostic rating index represented as the function of the signal intensity Mo of the first magnetic resonance signal received from the subject according to the first imaging condition unaccompanied by the application of the MT pulse, and the signal intensity Ms of the second magnetic resonance signal received from the subject according to the second imaging condition accompanied by the application of the MT pulse.

(MT Pulse)

The MT pulse is a radio wave used to induce cross-relaxation (magnetization transfer effect) between protons (bound water) bound to a high molecular weight compound such as body tissue and free water protons. The there are no particular limitations on the form of the MT pulse used in the present teaching provided it allows the obtaining of effective magnetization transfer effect in body tissue. For example, the MT pulse may have a sinc waveform or a waveform having a Gaussian distribution.

The MT pulse excites a frequency band separated by a prescribed frequency from the resonance frequency of protons contained in water. Although there are no particular limitations on the frequency used for excitation, the frequency is preferably near the resonance frequency of water. More specifically, the location from the resonance frequency of water (offset frequency) is preferably 20 ppm or less and more preferably 10 ppm or less.

(First Imaging Condition and Second Imaging Condition)

The signal intensity Mo of the first magnetic resonance signal and the signal intensity Ms of the second magnetic resonance signal used to calculate the lesion diagnostic rating index can be acquired by imaging tissue with the magnetic resonance imaging apparatus according to the first imaging condition unaccompanied by application of the MT pulse and the second imaging condition accompanied by application of the MT pulse. For example, in the case the first imaging condition executes scans based on imaging pulse sequences, the second imaging condition preferably radiates the MT pulse before the imaging sequence pulses. Furthermore, pulses other than the imaging pulses and MT pulse may also be added to the pulse sequences of the first imaging condition and the second imaging condition either simultaneous to or separate there from.

In the second imaging condition, there are no particular limitations on the form in which the MT pulse is radiated provided it is able to induce magnetization transfer effect. For example, the MT pulse may be composed as a single, independent RF pulse or composed of a plurality of cyclically repeated RF pulses. The MT pulse is preferably radiated in the form of a single RF pulse.

There are no particular limitations on the application time, application intensity, flip angle or bend width and the like of the MT pulse at this time, and can be suitably set corresponding to the target tissue or irradiated region.

Furthermore, there are no particular limitations on the form of the imaging pulse sequences, and although they may be two-dimensional scans or three-dimensional scans, three-dimensional scans are preferable. In addition, there are also no particular limitations on the form of the pulse train, and various types of pulse trains can be suitably employed, such as spin echo (SE), gradient echo (GRE), spoiled gradient recalled acquisition in steady state (SPGR), high-speed SPGR, field echo (FE) or high-speed SE. SPGR is used preferably.

In FIG. 1, an example of a pulse train used in imaging for calculating the lesion diagnostic rating index is shown. In FIG. 1, FIG. 1(a) indicates a pulse sequence set with the first imaging condition, while FIG. 1(b) indicates a pulse sequence set with the second imaging condition.

As shown in FIG. 1, in contrast to the pulse train being composed of imaging pulse sequences for gathering imaging data under the first imaging condition, under the second imaging condition, an MT pulse sequence is set so as to be applied to a subject in the form of a free pulse prior to gathering of imaging data. Namely, as shown in FIG. 1(b), under the second imaging condition, an MT pulse sequence is first executed followed by the execution of scans based on imaging pulse sequences.

Furthermore, there are no particular limitations on the configuration and the like of the magnetic resonance imaging (MRI) apparatus used for imaging provided it is configured to be able to apply an MT pulse. For example, this type of apparatus can be configured by adding a program and the like for setting a function that applies an MT pulse to an ordinary MRI apparatus commonly used in the routine clinical setting.

(Lesion Diagnostic Rating Index)

This diagnosis method detects a presage of a lesion in living tissue based on the lesion diagnostic rating index. The lesion diagnostic rating index is represented as a function of a signal intensity when not irradiating a region of interest with the MT pulse (the signal intensity Mo of the first magnetic resonance signal), and a signal intensity when the region of interest is irradiated with the MT pulse (the signal intensity Ms of the second magnetic resonance signal). Examples of functions (numerical formulas) that include the Mo and Ms that represent the lesion diagnostic rating index include any numerical formula selected from the group consisting of |Mo-Ms|, Mo/Ms and Ms/Mo as well as numerical formulas including one type or two or more types of numerical formulas selected from this group. The function is typically represented by the following equation (1). Namely, the lesion diagnostic rating index can be represented by the rate of decrease (%) of signal intensity represented by dividing the difference between Mo and Ms by the signal intensity during irradiation (the signal intensity Ms of the second magnetic resonance signal).

(Equation 2)

$$ECR(\%) = ((Mo-Ms)/Ms) \times 100 \qquad\qquad (1)$$

The lesion diagnosis rating index is preferably represented by equation (1) above. When the lesion diagnostic rating index is represented by equation (1) above, subtle changes in signal intensity between when radiating and not radiating the MT pulse can be amplified as compared with a magnetization transfer ratio (MTR) that represents signal intensity in terms of a rate of decrease. In other words, according to the lesion diagnostic rating index represented by equation (1) above, the portion affected by magnetization transfer effect can be emphasized. Furthermore, MTR conventionally refers to the rate of decrease (%) of signal intensity represented by dividing the difference between signal intensity when a region of interest is not irradiated with an MT pulse and signal intensity when a region of interest is irradiated with an MT pulse by the signal intensity when not irradiated with an MT pulse.

The lesion diagnostic rating index is able to quantitatively represent the effect of magnetization transfer effect. This magnetization transfer effect makes it possible to provide information that reflects intermolecular cross-relaxation between water and high molecular weight substances or proteins and the like, and is therefore considered to be able be used to evaluate minute structural changes in body tissue. Thus, the lesion diagnostic rating index is thought to be able to provide information for evaluating minute structural changes in tissue. More specifically, the lesion diagnostic rating index is thought to demonstrate elevated values in normal tissue in which fibrosis has not occurred due to a large magnetization transfer effect, and demonstrate low values in abnormal tissue exhibiting fibrosis due to a small magnetization transfer effect.

The lesion diagnostic rating index also makes it possible to detect lesions or abnormalities in body tissue earlier than blood or biochemical tests. This is thought to be because lesions or abnormalities can be determined at the stage of a presage prior to the appearance of tissue functional abnormalities, since the lesion diagnostic rating index is able to detect subtle changes in the molecular structure of body tissue.

Although there are no particular limitations on the subject region of body tissue from which the lesion diagnostic rating index is to be acquired, in the case the objective is to detect a presage, it is preferable to use normal tissue for the subject region in which abnormalities have not been detected in other diagnostic imaging or biopsy. The use of the lesion diagnostic rating index makes it possible to detect minute structural changes in the tissue as a lesion or presage thereof based on a decrease in the tissue diagnostic rating index even in tissue which at first may appear 'normal' according other testing information.

There are no particular limitations on the size of location and so forth of the region targeted for calculation of the lesion diagnostic rating index provided it is a measurement site that is identical or considered to be identical in terms of the first magnetic resonance signal and the second magnetic resonance signal. The size of the region, for example, may use one pixel or two or more pixels in images obtained according to the first imaging condition and the second imaging condition, or may be a prescribed area (for example, several to several tens of square millimeters) of an image or body tissue. In the case of calculating the lesion diagnostic rating index in a prescribed area of an image, for example, the lesion diagnostic rating index for each pixel contained in the prescribed area may be calculated followed by determination of the average value thereof.

(Mode for Detecting a Lesion in living tissue or Presage Thereof)

There are no particular limitations on the mode for detecting a lesion in living tissue or a presage thereof based on the lesion diagnostic rating index calculated in this manner, and various modes can be employed. Here, "detection of a lesion in living tissue or a presage thereof" at least includes the detection of the presence or absence of a presage of a lesion in body tissue. It may further include detection of the presence of a lesion, its disease state and its degree of progression.

One form for detecting a lesion in living tissue or presage based on the lesion diagnostic rating index consists of setting one type or two or more types of lesion diagnostic rating indices in the form of threshold information for detecting a presage from lesion diagnostic rating indices of a healthy subject group and a patient group ultimately definitely diagnosed with a disease related to the lesion in question, and then detecting the lesion or presage thereof based on the lesion diagnostic rating index. More specifically, a single lesion diagnostic rating index is set in the form of threshold information for which a lesion or a presage thereof is suspected in the target body tissue from the lesion diagnostic rating indices of a healthy subject group and a patient group. In addition to this, the presage is detected based on the difference between the lesion diagnostic rating index of a subject patient and the set threshold information. For example, the lesion diagnostic rating index in the form of threshold information for which the presage is suspected is set as an upper limit value. Namely, in the case a measured lesion diagnostic rating index is equal to or less than the fixed value, the presage can be detected by considering the measured lesion diagnostic rating index to be indicative of the presage of the lesion. In addition, a lesion diagnostic rating index in the form of threshold information that is considered to be normal and a lesion diagnostic rating index in the form of threshold information that is considered to be abnormal may both be set, and thereby a presage can be detected by considering the measured lesion diagnostic rating index to be indicative of the presage of a lesion when the lesion diagnostic rating index is within the range of 'normal to abnormal'. Threshold information may also be set by further dividing the range of 'normal to abnormal'.

Another form of detecting a lesion in living tissue or presage thereof based on the lesion diagnostic rating index consists of using changes in the lesion diagnostic rating index as a function of time from before diagnosis to after diagnosis (accompanying progression of a disease) obtained from a patient group for which a definitive diagnosis of the disease has already been made. In this form, diagnosis is made based on set threshold information by using lesion diagnostic rating index data of a subject disease tissue of the patient group, and setting a lesion diagnostic rating index in the form of threshold information for assessing the presage, presence and degree of lesion progression of a tissue lesion from that data.

For example, a prescribed rate of decrease based on the amount of change (versus time) of the lesion diagnostic rating index of the patient group can be used as threshold information for detecting a presage of a tissue lesion and detecting the presence of a tissue lesion. In particular, the rate of decrease of the lesion diagnostic rating index prior to diagnosis is preferable as the threshold information for detecting the presage of the tissue lesion, the rates of decrease of the lesion diagnostic tissue rating before and after diagnosis are preferable as the threshold information for detecting the presence of the tissue lesion, and the rate of decrease of the lesion diagnostic rating index after diagnosis is preferable as the threshold information for assessing the degree of progression of the tissue lesion.

Furthermore, another form for detecting the tissue lesion or the presage thereof includes a form in which the lesion is detected based on rates of decrease (decreasing tendency) over time of the lesion diagnostic rating index without being particularly dependent on an amount of change in the lesion diagnostic rating index in the form of threshold information. According to this form, the progression of a disease and the like of the target region can be assessed. In addition, the accuracy of the diagnosis can be further enhanced in comparison with the case of assessing the presence or degree of progression of the lesion only on the basis of the value of the lesion diagnostic rating index at a given point in time. Namely, in the case of observing the lesion diagnostic rating index of the same patient over time, it is thought that the larger the degree of decrease in the lesion diagnostic rating index at the same region of interest, the greater the structural change in the molecules of that region. Thus, in a region where the rate of decrease of the lesion diagnostic rating index is large, the lesion can be judged to have a high possibility of progressing rapidly. Thus, monitoring the lesion diagnostic rating index is useful for early diagnosis and early treatment.

In addition, the lesion diagnostic rating index can be set in the form of threshold information for detecting the presage of the tissue lesion, in the form of threshold information for detecting the presence of the tissue lesion, and in the form of threshold information for respectively diagnosing the degree of progression of a disease, from lesion diagnostic rating index data of before diagnosis, during diagnosis and after diagnosis of a patient group. In this form, the lesion diagnostic rating index in the form of threshold information for which a presage or presence of a lesion is suspected is set in as the upper limit value. In addition, the amount of change in the lesion diagnostic rating index is set in the form of a lower limit value. For example, when the lesion diagnostic rating index is equal to or less than the fixed value or below the fixed value, the lesion diagnostic rating index can be detected as indicating the presage of a tissue lesion. Further, when an amount of change in the lesion diagnostic rating index is equal to or greater than the fixed value or exceeds the fixed value, the lesion diagnostic rating index can be detected as indicating the presage of a tissue lesion.

In setting threshold information with respect to the lesion diagnostic rating index in this manner, there are no particular limitations on the procedure for making a definitive diagnosis for the patient group provided it is that other than the lesion diagnostic rating index. For example, a blood test, urinalysis, imaging test or pathology test and the like can be used separately or in the form of suitably combinations thereof. An example of a preferable technique for making a definitive diagnosis is testing involving various types of clinical laboratory testing. By using information obtained from testing in this manner (testing information), statistically significant threshold information can be set by using a correlation with the lesion diagnostic rating index.

For example, as shown in FIG. 2, a graph can be prepared of the correlation between lesion diagnostic rating index data and prescribed testing information for a healthy subject group and a patient group, and the lesion diagnostic rating indices can be set in the form of threshold information based on the lesion diagnostic rating indices when equivalent to a boundary value (upper limit or lower limit of an abnormal range) of the testing information. Namely, a lesion diagnostic rating index for tissue being normal can be set as threshold information E1 based on the lesion diagnostic rating indices of the healthy subject group, and a lesion diagnostic rating index when equivalent to the boundary value of the testing information can be set for the patient group as threshold information E2. Furthermore, the threshold information E2 is effective for detecting the presence of a tissue lesion. For example, when a calculated lesion diagnostic rating index exceeds the threshold information E2 but is equal to or less than the threshold information E1, the lesion diagnostic rating index can be considered to indicate the presence of the presage of a tissue lesion. In addition, when the calculated lesion diagnostic rating index is less than or equal to the threshold information E2, the lesion diagnostic rating index can be considered to indicate the presence of a lesion.

Particularly, the lesion diagnostic rating index for assessing a future patient group for which the presage of a tissue lesion is present can be set as threshold information E3 from the graph of lesion diagnostic rating indices of the healthy subject group and the patient group. For example, as for the threshold information E3, when the lesion diagnostic rating index is higher than the threshold information E3, the lesion diagnostic rating index can be considered to indicate the absence of the presage of a tissue lesion, and when it is equal to or below the threshold information E3, the lesion diagnostic rating index can be considered to indicate the presence of the presage of a tissue lesion.

An example of threshold information of the lesion diagnostic rating index is shown in FIG. 3. In FIG. 3, tissue is evaluated as normal when the lesion diagnostic rating index exceeds the threshold information E1, is evaluated as indicating the presence of the presage of a tissue lesion when it exceeds the threshold information E2 but is equal to or less than the threshold information E1, and is evaluated as indicating the presence of the tissue lesion when it is equal to or less than the threshold information E2. Furthermore, although the state of the lesion is evaluated to one of three stages in FIG. 3, the present teaching is not limited thereto, but rather it may also be evaluated to one of two stages, or alternately to one of four stages or more.

For example, the threshold information E2 may be set with a value within the range of 70% to 90%. The threshold information E2 is more preferably set to a value in the vicinity of 80%, and even more preferably is set to a value in the vicinity of 80% in the case of applying to a liver disease.

When setting a logical expression and the like based on a prescribed value selected for use as threshold information during detection of the tissue lesion and the like using the lesion diagnostic rating index, whether or not that prescribed value is to be included can be set as is suitable. Thus, in the present specification, when detecting the presence of a tissue lesion or normalcy in the tissue based on the threshold information, determination of being equal to or greater than the prescribed threshold information may be substituted with determination of whether the prescribed threshold information is exceeded. In addition, determination of equal to or less than the prescribed threshold information may similarly be substituted with determination of being less than the prescribed threshold information.

Although there are no particular limitations on the testing information for setting the threshold information, it is preferable to use testing information related to a biological factor that increases or decreases with the progression of the lesion to be diagnosed. For example, testing information relating to blood, urine, stool, saliva, sputum or cell density and the like can be used. Testing information relating to blood is preferable.

In the case of using testing information relating to blood, there are no particular limitations on the type thereof. For example, in the case the body tissue is liver tissue, various hematological data that serves as an indicator of liver function can be used, such as glutamic oxaloacetic transaminase (GOT), glutamic pyruvic transaminase (GPT), albumin, bilirubin, cholinesterase, indocyanine green (ICG), lactate dehydrogenase (LDH), prothrombin time (PT), $\gamma$-GTP, alkaline phosphatase (ALP), $\alpha$-fetoprotein (AFP), abnormal prothrombins, carcinoembryonic antigen (CEA) or CA19-9. The hematological data is preferably one type or two or more types of data selected from the group consisting of GOP, GPT, albumin, bilirubin, cholinesterase and ICG, more preferably one type or two or more types of hematological data selected from the group consisting of albumin, bilirubin and cholinesterase, and even more preferably albumin and/or cholinesterase. In addition, platelet count may also be used as hematological data.

The lesion diagnostic rating index data is preferably indicated in a state able to be visualized on an image. This enables the presence of a presage or a presence of a tissue lesion in a targeted region to be accurately determined. Visualization can be carried out by, for example, displaying each region of lesion diagnostic rating indices divided according to threshold information of the lesion diagnostic rating indices by distinguishing their hue, saturation or contrast and the like.

Another form of detecting a lesion in living tissue or presage thereof based on the lesion diagnostic rating index consists of using a correlation between testing information other than the lesion diagnostic rating index obtained from a healthy subject group and a patient group for which a definitive diagnosis has been made by a method differing from diagnosis using the lesion diagnostic rating index and the lesion diagnostic rating index correlated with the testing information, as well as carrying out detection based on the testing information of a subject other than the lesion diagnostic rating index and the lesion diagnostic rating index. Namely, the correlation between testing information and lesion diagnostic rating index data is used. The testing information is related to a tissue lesion or disease other than the lesion diagnostic rating index that is obtained from the healthy subject group and the patient group in which the prescribed disease has ultimately been confirmed,. As a result, the certainty of diagnosis of the subject disease can be increased. In this form, as shown in FIG. 4, for example, a graph of the distribution of a healthy subject group (correlation diagram thereof) and the distribution of a patient group (correlation diagram thereof) can be used by plotting testing information on the horizontal axis and plotting lesion diagnostic rating indices on the vertical axis for the healthy subject group and the patient group. Namely, when the lesion diagnostic rating index and testing information have been acquired for a patient to be diagnosed, whether or not a point based on this lesion diagnostic rating index and testing information falls within either distribution (correlation diagram thereof) can be assessed based on statistical techniques. According to this technique, a lesion having a high probability of appearing in the future, namely a lesion or presage thereof, can be detected even if testing information does not exhibit abnormal values.

There are no particular limitations no the testing information used in this form. For example, testing information relating to various biological factors such as blood, urine, stool, sputum or saliva, information relating to pathological tests, or information relating to diagnostic imaging such as CT or ultrasound tests can be used. Testing information used when setting the threshold information is used preferably. Testing information at least relating to blood is used more preferably.

When the lesion diagnostic rating index is calculated according to the equation (1), it is preferable for being able to quantitatively evaluate a lesion, presage thereof or progression of a disease. In addition, since the evaluation is a quantitative evaluation, minute lesions or presages thereof of a degree that cannot be seen by ordinary diagnostic imaging can be determined, and this leads to a decrease in the rate of false negatives. Moreover, differing from testing information such as hematological data, the region where the lesion has appeared can be specified. Moreover, since evaluations can be made non-invasively, diagnoses can be made at affected regions where biopsy is inappropriate and there is less of a burden on patients.

In addition, according to the lesion diagnostic rating index, the tissue lesion or the presage thereof can be detected even in tissue that has been diagnosed as being normal on the basis of testing information and pathology information such as clinical laboratory data or image information other than the lesion diagnostic rating index. More specifically, in addition to being able to diagnose the tissue lesion or presage thereof in tissue that has been tentatively diagnosed as being normal tissue, in the case a portion of an organ has been diagnosed as abnormal, the presage can be detected in the case the lesion has progressed to tissue tentatively diagnosed as 'normal' that has yet to be diagnosed as abnormal. In addition, by investigating the lesion diagnostic rating index of an organ that has yet to be diagnosed with cancer of a cancer patient, a presage of metastasis to that organ can also be detected. In addition, by following the lesion diagnostic rating index of an organ that has been completely cured over time, a presage of recurrence can also be detected. Thus, by detecting the tissue lesion or presage thereof based on the lesion diagnostic rating index, preventive medicine or early treatment can be performed in anticipation of the appearance of the tissue lesion.

Furthermore, although diagnosis using the lesion diagnostic rating index as described above can demonstrate adequate effectiveness even by examining the lesion diagnostic rating index at a fixed point in time, it is able to demonstrate even greater effectiveness and improve the accuracy of detecting presages preferably by periodic monitoring thereof. Thus, both examination of the lesion diagnostic rating index and monitoring examination thereof can greatly contribute to early diagnosis and early treatment by using in examinations conducted e.g., in health examinations, examinations conducted prior to making definitive diagnoses, examinations during the course of monitoring the progress of a patient such as during relapse, metastasis or recurrence, examinations conducted during the course of treatment, examinations conducted for the purpose of determining prognoses, or examinations conducted following relapse or recurrence.

The present diagnosis method may also be realized manually or may be realized with one or a plurality of computers. In the case of realizing by computer, the computer may be composed separately from the magnetic resonance imaging apparatus or may compose a portion of the magnetic resonance imaging apparatus.

The following provides an explanation of the magnetic resonance imaging apparatus of the present teaching with reference to FIG. 5. This apparatus is suitable for executing the previously described diagnosis method of the present teaching.

(Magnetic Resonance Imaging Apparatus)

The magnetic resonance imaging apparatus (to also be abbreviated as MRI) 10 of the present teaching has a module capable of applying an MT pulse, a module for calculating the lesion diagnostic rating index, and a module for detecting a lesion or presage thereof based on the lesion diagnostic rating index, added to an MRI apparatus used in the routine clinical setting. Furthermore, the MT pulse is has been previously explained with regard to the diagnosis method of the present teaching. As shown in FIG. 5, this apparatus is provided with a scanner 20 that executes scans based on pulse sequences, and a controller 30 that controls the scanner 20 and carries out processing of image data.

(Scanner)

As shown in FIG. 5, the scanner 20 is provided with a static magnetic field generation section 22 that generates a static magnetic field in an imaging space, a gradient magnetic field generation section 24 that generates a gradient magnetic field for adding location information to the static magnetic field, a transmission/reception module 26 that transmits an RF signal to a subject and receives an MR signal from the subject, and a bed section 28 on which the subject lies down.

The static magnetic field generation section 22 forms the static magnetic field in an imaging space in which the subject is contained. There are no particular limitations on the form of the static magnetic field generation section 22, and for example, may be composed of a static magnetic field magnet and a static magnetic field power supply or composed of a permanent magnet. In addition, although there are limitations on the direction in which the static magnetic field is formed, it is preferably formed so that the direction of the static magnetic field is in line with a direction perpendicular to the axial direction of the subject's body.

The gradient magnetic field generation section 24 adds location information to the MR signal from the subject by applying a gradient to the magnetic field strength of the imaging space formed by the static magnetic field. The gradient magnetic field generation section 24 can use an ordinary gradient magnetic field coil composed of three pairs of coils, corresponding to the three directions of the x direction, y direction and z direction, inside a primary magnet.

The transmission/reception module 26 is composed of an RF coil, for example, and carries out transmission and reception of electromagnetic signals. Namely, the transmission/reception module 26 transmits an RF pulse to the subject arranged within the imaging space formed by the static magnetic field. As a result, a high-frequency magnetic field is formed and the spin of protons in the imaged region of the subject is excited. Electromagnetic waves emitted from the excited protons are then received from the subject in the form of the magnetic resonance signal (MR signal).

The bed section 28 is a bed that allows the subject to lie down thereon. The bed section 28 is configured so as to be able to move between the inside and outside of the imaging space.

(Controller)

The controller 30 is provided with an imaging condition setting module32 that allows setting of the first imaging condition and the second imaging condition, an image generation module 34 that generates images, a calculation module 36 that calculates the lesion diagnostic rating index, a detection module 38 that detects a presage or presence of a lesion of body tissue based on the calculated lesion diagnostic rating index, and a control module 40 that controls operation of the entire controller 30 and each section of the scanner 20.

The imaging condition setting module32 sets an imaging condition based on input data input to an input section 33 into which instructions of an operator have been input (such as a number of scans, flip angle, number of echoes, echo time and other scan parameters and imaging commands). Namely, pulse sequences to be executed by the scanner 20 are generated based on the input data. This imaging condition setting module32 at least allows the setting of a first imaging condition that is unaccompanied by application of an MT pulse and a second imaging condition that is accompanied by the application of the MT pulse.

The image generation module 34 carries out image processing based on the MR signal input from the transmission/reception module 26 to the control module 40. Namely, the image generation module 34 arranges digital data from the transmission/reception module 26 in a Fourier space (k-space), and reconstructs the data to image data of real space by applying two-dimensional or three-dimensional Fourier transformation. There are no particular limitations on the form of image processing by the image regeneration section 34, examples of which include addition processing among multiple frames of image data (such as simple addition processing, averaging processing or weighted addition processing), difference operation processing and maximum intensity projection (MIP) processing. Images reconstructed in this manner are displayed on a display section 35. In addition, information that correlates threshold information of the lesion diagnostic rating indices with colors is stored in the image generation module 34, and the image generation module 34 carries out imaging based on the values of the lesion diagnostic rating index for each pixel.

The calculation module 36 calculates the lesion diagnostic rating index based on a preliminarily stored program. More specifically, image data imaged under the first imaging condition and image data imaged under the second imaging condition are input from the control module 40, the lesion diagnostic rating index is calculated, and the calculated lesion diagnostic rating index is output to the control module 40.

The detection module 38 detects the presage or the presence of a lesion in living tissue based on the lesion diagnostic rating index calculated in the calculation module 36. This detection module 38 stores preset threshold information of the lesion diagnostic rating index (such as the threshold information shown in FIG. 3), and detects the presence of the presage or the presence of a tissue lesion, or assesses the disease state or degree of progression thereof, based on this threshold information. Furthermore, a setting method used in the previously described diagnosis method of the present teaching can be applied as is for setting the threshold information of the lesion diagnostic rating index.

The control module 40 controls the scanner 20 so that scans are executed based on pulse sequences set with the imaging condition setting module32. The control module 40 is configured, for example, in the form a microprocessor having a CPU 42 as the core thereof, and control operation of each section that composes the scanner 20 as well as the entire controller 30. Namely, the control module 40 is electrically connected to the static magnetic field generation section 22, the gradient magnetic field generation section 24, the transmission/reception module 26 and the bed section 28, outputs control signals and the like to each of these sections, and inputs MR signals and the like from the transmission/reception module 26. In addition, the control module 40 is also electrically connected to the imaging condition setting module32, the input section 33, the image generation module 34, the display section 35, the calculation module 36 and the detection module 38 that compose the controller 30, and carries out exchange of control signals, command signals and the like.

(Operating Method)

Next, an explanation is provided of the functions of the magnetic resonance imaging apparatus 10 of the present teaching configured in this manner, and particularly a module for detecting the lesion in living tissue or the presage thereof using the lesion diagnostic rating index as an indicator. The CPU 42 of the control module 40 executes the lesion diagnostic rating index indicator diagnosis routine shown in FIG. 6 when a scan start command is input from the input section 33.

When this lesion diagnostic rating index indicator diagnosis routine is started, the CPU 42 of the control module 40 first executes a scan based on the first imaging condition set with the imaging condition setting module32 (Step S100). More specifically, together with forming a magnetic field in the imaging space by driving the static magnetic field generation section 22 and the gradient magnetic field generation section 24 corresponding to a preset pulse train, the transmission/reception module 26 is driven so that a scan is executed based on an ordinary imaging sequence in which an MT pulse is not applied. As a result, a first magnetic resonance signal is obtained that is not affected by magnetization transfer effect.

When all scans based on imaging sequences have been completed, the CPU 42 carries out three-dimensional Fourier transformation on k-space digital data that has been gathered and arranged as a result of imaging under the first imaging condition, and reconstructs that data into real space image data (Step S 110).

Continuing, the CPU 42 executes a scan based on the second imaging condition set with the imaging condition setting module32 (Step S120). Under the second imaging condition, together with driving the static magnetic field generation section 22 and the gradient magnetic field generation section 24, the transmission/reception module 26 is driven to that an MT pulse and an imaging pulse are applied to a subject on the bed section 28. As a result, an MR signal that reflects a magnetization transfer effect (second magnetic resonance signal) can be obtained from the subject. When all scans based on imaging sequences having an MT pulse added to the leading end thereof have been completed, k-space digital data that has been gathered and arranged is reconstructed to real space image data in the same manner as the first imaging condition (Step S 130).

When imaging based on the first imaging condition and the second imaging condition has been completed, the CPU 42 detects the lesion diagnostic rating index using the signal intensity Mo of the first magnetic resonance signal and the signal intensity Ms of the second magnetic resonance signal (Step S140). Here, calculation processing is carried out for each corresponding pixel.

After having calculated the lesion diagnostic rating index data, the CPU 42 detects the presage and the presence of a lesion in living tissue based on the calculated lesion diagnostic rating index data and displays the result on the display section 35 (Step S150), thereby completing this routine. In the detection step of Step S 150, together with comparing the calculated lesion diagnostic rating index data of each pixel with a corresponding relationship with threshold information and saturation of preliminarily stored lesion diagnostic rating indices, specifying a color corresponding to the lesion diagnostic rating index data for each pixel, and displaying an image composed of each specified color on the display section 35, information that correlates tissue state with color is displayed on the display section 35. As a result, since the lesion diagnostic rating index data is visualized in a state that is correlated with the state of the tissue, an operator who has viewed the display section 35 can easily and accurately determine the lesion or presage thereof and the disease state.

Furthermore, although detection results are displayed by imaging the lesion diagnostic rating index in Step S 150 in the present embodiment, if information relating to a lesion in the manner of the region where the lesion is present or the degree of progression thereof is displayed, the detection results may be displayed by a method other than imaging the lesion diagnostic rating index (such as by using characters or numerical values).

In addition, in Step S 140, the lesion diagnostic rating index may be calculated from signal intensity of a region of interest selected by the operator on an image imaged according to the first imaging condition or the second imaging condition. At this time, the presage of a lesion may be detected in that region of interest in Step S 150.

Moreover, although a tissue lesion or presage thereof was detected in Step S150 based on the lesion diagnostic rating index at a fixed point in time, instead of or in addition thereto, the amount of change (versus time) in lesion diagnostic rating indices may be calculated based on a preliminarily stored past lesion diagnostic rating index of the same patient and the current calculated lesion diagnostic rating index, and the degree of progression or metastasis of a lesion may also be analyzed based on the calculated amount of change.

Furthermore, the functions, applications and the like of the previously explained diagnosis method of the present teaching can be directly applied to the magnetic resonance imaging apparatus and operating method thereof of the present teaching. Thus, the present teaching includes each of various forms for realizing each of the forms of the diagnosis method of the present teaching.

(Diagnostic Imaging System)

The diagnostic imaging system of the present teaching is provided with a transmission/reception module, a control module, a calculation module and a detection module in the previously described magnetic resonance imaging apparatus of the present teaching. The configuration and functions and so forth of the previously explained diagnosis method of the present teaching, magnetic resonance imaging apparatus and operating method thereof can be directly applied to this diagnostic imaging system. Thus, the present teaching includes each of various forms for realizing each of the above-mentioned forms of the diagnosis method, the magnetic resonance imaging apparatus and the operating method thereof of the present teaching.

(Lesion in Living Tissue Detection Apparatus)

The detection apparatus of the present teaching can be provided with a module for acquiring the lesion diagnostic rating index represented by the equation (1) using the signal intensity Mo of the first magnetic resonance signal received from an imaged region of a subject according to a first imaging condition unaccompanied by the application of the MT pulse and the signal intensity Ms of the second magnetic resonance signal received from the imaged region of the subject according to the second imaging condition accompanied by the application of the MT pulse, and a detection module for detecting the lesion in living tissue or the presage thereof in the imaged region based on the lesion diagnostic rating index.

According to this detection apparatus, the lesion diagnostic rating index represented by the equation (1) can be acquired by inputting the signal intensities Mo and Ms acquired by a nuclear magnetic resonance imaging apparatus. Moreover, a lesion or presage thereof in the imaged region can be detected based on the acquired lesion diagnostic rating index. According to this detection apparatus, by acquiring the above-mentioned signal intensities from a conventional nuclear magnetic resonance imaging apparatus, and calculating the lesion diagnostic rating index or acquiring the lesion diagnostic rating index directly, the lesion in living tissue or presage thereof that was unable to be detected solely with a conventional nuclear magnetic resonance imaging apparatus can be easily detected.

The module for acquiring the lesion diagnostic rating index may be a module for inputting the lesion diagnostic rating index calculated on the basis of the equation (1) in a nuclear magnetic resonance imaging apparatus and the like, or may be a module for calculating the lesion diagnostic rating index based on the equation (1) within the present detection apparatus based on the signal intensities Mo and Ms input into the detection apparatus after having been acquired by a nuclear magnetic resonance imaging apparatus and the like. A module for executing a step in which the lesion diagnostic rating index is acquired with the previously explained nuclear magnetic resonance imaging apparatus can be employed for this module.

The detection module for detecting the lesion or presage thereof provided in this detection apparatus can employ a module for executing a step in which the lesion in living tissue or presage thereof is detected in the nuclear magnetic resonance imaging apparatus. In addition, each of type of embodiment of this module can also be applied directly to the present detection apparatus.

Moreover, the present detection apparatus can be provided with an imaging module for imaging the degree of the lesion in the imaged region based on the lesion diagnostic rating index of that imaged region. The degree of the lesion of the imaged region, namely the degree of progression from the lesion or the presage thereof, can be easily determined by imaging the degree of the lesion in the imaged region based on the lesion diagnostic rating index. It is necessary to preliminarily correlate the degree of the lesion with lesion diagnostic rating indices based on the detection module in order to output an image of the degree of the lesion in the imaged region based on the lesion diagnostic rating index. Here, time-based changes in the lesion diagnostic rating index, threshold information of the lesion diagnostic rating index and the correlation (graph) between testing information and the lesion diagnostic rating index can be used to correlate the degree of the lesion with the lesion diagnostic rating index as previously explained.

The imaging module is only required to form image information obtained by imaging the degree of a lesion. Thus, the formed image information may be output directly a suitable external image display module such as a monitor or nuclear magnetic resonance imaging device, or in the case the present detection apparatus is provided with an image display module, the image may be displayed on the image display module.

(Program)

According to the present teaching, a diagnostic imaging program is provided for detecting the lesion in living tissue or the presage thereof based on the lesion diagnostic rating index. The diagnostic imaging program of the present teaching is a program that executes the detection step of the above-mentioned operating method in one or a plurality of computers. This program may be recorded on a computer-readable recording medium (such as a hard disc, ROM, FD, CD or DVD), may be distributed to a different computer from a certain computer through a transfer medium (such as the Internet, LAN or other communication network), or may be received by any other mode. Effects similar to the above-mentioned operating method are obtained by executing this program in a single computer or by executing in a plurality of computers by assigning respective operations thereof to each computer.

Although the following provides an explanation of the present teaching through specific examples thereof, the present teaching is not limited to the specific examples described below.

(Example 1)

(MRI)

Table 1 shows the results of imaging the livers of 17 patients (consisting of 3 patients with normal livers, 3 patients with metastatic liver cancer and 11 patients with hepatocellular carcinoma) using a magnetic resonance imaging apparatus. Definitive diagnosis of the patients shown in Table 1 was made by suitably combining techniques other than the lesion diagnostic rating index, such as MRI and CT imaging testing, blood testing and the like. Furthermore, among the disease names shown in Table 1, HCC indicates hepatocellular carcinoma, TAE indicates transcatheter arterial embolization, LC indicates liver cirrhosis and FNH indicates focal nodular hyperplasia.

[Table 1]

| Case | Age | Gender | Height (m) | Weight (kg) | BMI | Disease name | Child-Pugh classification |
|------|-----|--------|------------|-------------|-----|--------------|---------------------------|
| 1 | 58 | M | 1.665 | 66.2 | 23.88 | HCC, postTAE | B |
| 2 | 58 | M | 1.665 | 66.2 | 23.88 | HCC, postTAE | B |
| 3 | 61 | M | -- | -- | -- | Suspected FNH | A |
| 4 | 63 | M | 1.694 | 79.5 | 27.70 | HCC | A |
| 5 | 71 | F | 1.514 | 57.6 | 25.13 | Colorectal cancer w/liver metastasis | A |
| 6 | 67 | F | 1.445 | 56.2 | 26.92 | HCC,LC | B |
| 7 | 49 | M | 1.660 | 79.0 | 28.67 | Bladder cancer | A |
| 8 | 60 | M | 1.695 | 65.6 | 22.83 | Lung cancer w/liver metastasis | A |
| 9 | 66 | M | 1.626 | 63.6 | 24.06 | HCC, LC | C |
| 10 | 79 | M | 1.647 | 75.8 | 27.94 | Colon cancer w/liver metastasis | A |
| 11 | 46 | F | 1.490 | 44.0 | 19.82 | Suspected pancreatic tumor | A |
| 12 | 71 | M | 1.720 | 80.6 | 27.24 | Suspected pancreatic tumor | A |
| 13 | 75 | F | 1.520 | 50.0 | 21.64 | HCC | B |
| 14 | 74 | M | 1.620 | 54.0 | 20.58 | HCC recurrence | B |
| 15 | 62 | F | -- | -- | -- | HCC | -- |
| 16 | 67 | M | -- | -- | -- | HCC | -- |
| 17 | 67 | M | -- | -- | -- | HCC, postOP | I -- |

A 1.5 T superconducting MR apparatus (GE, SIGNA MR/i 1.5 T High Speed Ver. 9.0) was used for the MR apparatus. Furthermore, this apparatus was installed with a program enabling radiation of MT-RF pulses. Imaging with this MR apparatus was carried out using 3D-spoiled gradient recalled acquisition in steady state (SPGR) without applying an MT-RF pulse, and MT-SPGR in which the MT-RF pulse was applied prior to SPGR.

The imaging conditions consisted of a repeat time TR of 39 msec, echo time TE of 6.9 msec, flip angle of 30°, matrix size of 256 x 96, FOV of 40 cm, slice thickness of 10 mm, overlap location of 0 mm, and location per slab of 8. In addition, the MT-RF pulse employed a frequency 7 ppm away from the resonance frequency of water to the side of the lower magnetic field for the offset frequency, and the pulses were radiated for 18 msec in the form of a sinc waveform (radiation intensity: 3.26 μT).

(Calculation of Lesion Diagnostic Rating Index)

Lesion diagnostic rating indices were calculated using image data obtained by imaging by SPGR and imaging by MT-SPGR. More specifically, the same region of an image of the same cross-section was extracted, the lesion diagnostic rating index was calculated for each pixel of the extracted region (region of interest) using the equation (1), and the average value of each pixel in that region was determined. Furthermore, a region judged to be normal tissue by MR was selected when extracting the region of interest on the images. The results are shown in Table 2.

[Table 2]

| Case | ECR | ICG | GOP | GPT | CHE | Albumin | Bilirubin |
|------|-----|-----|-----|-----|-----|---------|-----------|
| 1 | 92.1±16.8 | -- | 68 | 37 | 183 | 3.6 | 0.5 |
| 2 | 82.8±12.4 | 3.2 | 48 | 66 | 211 | 4.4 | 0.5 |
| 3 | 95.8±6.7 | -- | 20 | 29 | 199 | 4.7 | 0.3 |
| 4 | 81.3±6.9 | 17.1 | 74 | 70 | 152 | 4.0 | 0.9 |
| 5 | 92.7±8.6 | -- | 20 | 11 | 122 | 3.9 | 1.1 |
| 6 | 68.3±12.5 | 28.2 | 65 | 75 | 91 | 3.2 | 1.6 |
| 7 | 115.4±20.6 | -- | 30 | 53 | 178 | 4.6 | 0.4 |
| 8 | 92.5±6.5 | 4.4 | 18 | 16 | 182 | 4.1 | 0.7 |
| 9 | 68.0±13.3 | -- | 58 | 24 | 37 | 2.8 | 2.4 |
| 10 | 91.0±20.0 | 18.7 | 28 | 19 | 146 | 4.2 | 0.5 |
| 11 | 94.0±16.2 | -- | 17 | 13 | 116 | 3.8 | 0.4 |
| 12 | 98.0±13.5 | -- | 17 | 16 | 161 | 4.1 | 0.3 |
| 13 | 74.1±6.3 | -- | 101 | 92 | 101 | 3.7 | 1.2 |
| 14 | 82.7±8.2 | -- | 35 | 30 | 131 | 3.9 | 0.8 |
| 15 | 84.4±8.5 | 14.5 | 32 | 34 | 193 | 4.3 | 0.6 |
| 16 | 75.0±6.6 | 21.6 | 57 | 65 | 73 | 3.6 | 1.0 |
| 17 | 77.1±4.5 | 21.7 | 90 | 84 | 123 | 4.0 | 1.2 |

(Acquisition of Blood Data)

Blood tests were carried out on the patients shown in Table 1. The tests were carried out for the six parameters shown in Table 2 according to ordinary techniques used in the routine clinical setting. The results are shown in Table 2.

(Evaluation of Correlation Coefficients and Setting of Threshold Information for Lesion Diagnostic Rating Indices)

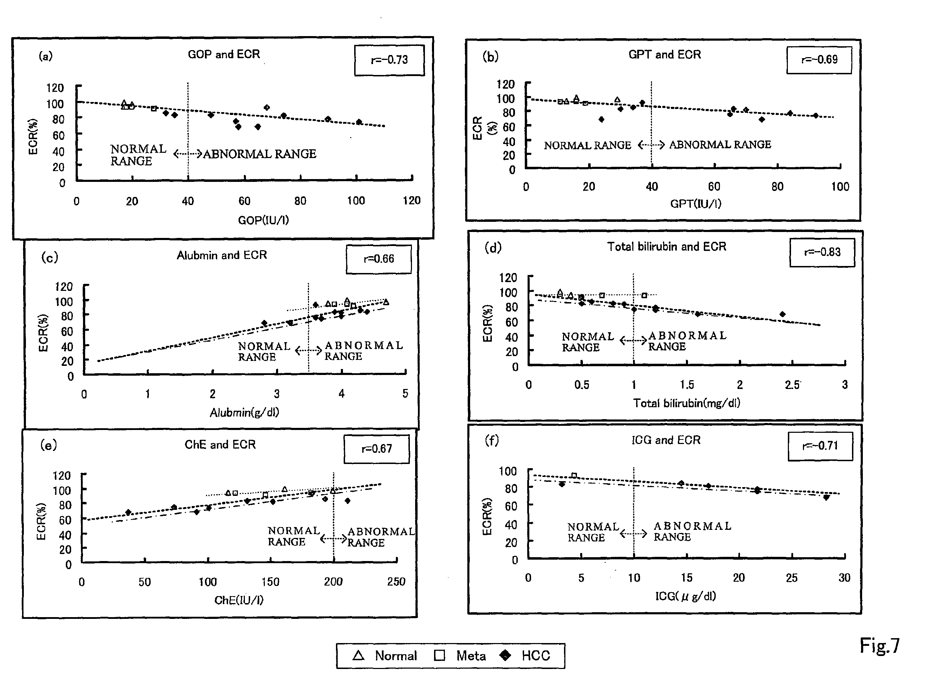

In addition to evaluating correlation coefficients between lesion diagnostic rating index data and blood data acquired from the patients, threshold information was set for the lesion diagnostic rating indices. In evaluating the correlation coefficients, a graph was prepared of correlation coefficients between the lesion diagnostic rating index data and each parameter of blood data of the patients as shown in Table 2, and the results were evaluated by calculating Spearman's rank correlation coefficient (r). In addition, setting of threshold information was carried out by determining the lesion diagnostic rating index equivalent to a boundary value for which each parameter of blood data was considered to be abnormal using the previously prepared graph. The resulting graphs and Spearman's rank correlation coefficients are shown. Furthermore, in FIG. 7, FIG. 7(a) indicates GOP (also AST (aspartate aminotransferase)) for the blood data, FIG. 7(b) indicates GPT (also ALT (alanine aminotransferase)), FIG. 7(c) indicates albumin, FIG. 7(d) indicates bilirubin, FIG. 7(e) indicates cholinesterase, and FIG. 7(f) indicates ICG.

As shown in FIG. 7, Spearman's rank correlation coefficients were -0.73 for GOP, -0.69 for GPT, 0.66 for albumin, -0.83 for bilirubin, 0.67 for cholinesterase and -0.71 for ICG. Strong correlations were observed between both parameters for GOP, bilirubin and ICG in particular. In addition, the lesion diagnostic rating indices equivalent to the boundary values of the blood data were determined to yield values in the proximity of 80% for each of FIG. 7(a) through 7(f) (to be referred to as the respective boundary values). Moreover, among FIG. 7(a) through 7(f), the distribution of a normal liver group and abnormal liver group were clearly distinguished for albumin in FIG. 7(c), bilirubin in FIG. 7(d) and cholinesterase in FIG. 7(e). In other words, in a comparison of lesion diagnostic rating indices when blood data is in the normal range, lesion diagnostic rating indices in the abnormal liver group were distributed in a lower range than those in the normal liver group.

On the basis of the above results, lesion diagnostic rating indices were determined to exhibit a strong correlation with blood data, and the values were observed to decrease as blood data became worse. In addition, liver function disorders were determined to be able to be detected when using values in the vicinity of 80% for the threshold values of the lesion diagnostic rating indices. Moreover, in the case albumin, bilirubin or cholinesterase was used for the blood data, since results were obtained for which the distributions (graphs) of the normal liver group and abnormal liver group were able to be distinguished, it was determined that a region of interest could be evaluated as normal or abnormal by evaluating to which distribution (graph) the lesion diagnostic rating index of that region belonged. Moreover, since the lesion diagnostic rating indices of the abnormal liver group were distributed in a range lower than that of the normal liver group despite blood data being in the normal range, lesion diagnostic rating indices in the abnormal liver group were suggested to exhibit values capable of being distinguished from the normal liver group at an earlier stage than blood data.

On the basis of these findings, the lesion diagnostic rating index was suggested to be able to serve as a novel indicator for detecting tissue abnormalities. In addition, the use of the lesion diagnostic rating index was suggested to enable earlier detection of a lesion than that based on blood test results. Moreover, when considering that the tissue diagnostic rating indices of the abnormal liver group yielded values capable of being distinguished from those of the normal liver group even when blood data was within the normal group, and that the lesion diagnostic rating index is considered to allow detection of microstructural changes in tissue, the lesion diagnostic rating index was suggested to be an extremely useful indicator for detecting and diagnosing not only the presence of a tissue lesion, but also a presage thereof.

(Example 2)

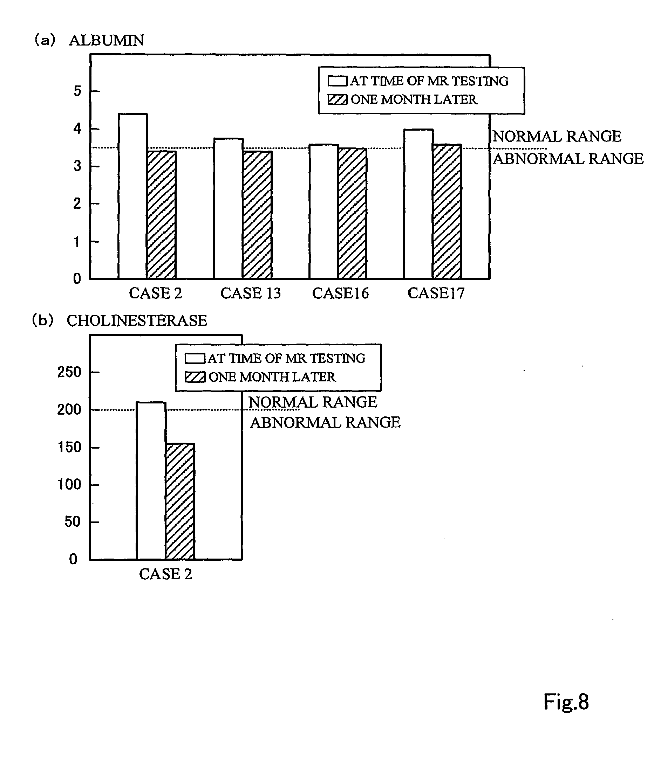

In Example 2, lesion diagnostic rating indices were calculated for those patients listed in Table 1 in which blood data was generally within the normal range and lesion diagnostic rating indices were in the vicinity of boundary values, followed by evaluating changes in blood data over time. Evaluations were carried out by comparing blood data at the time of calculating the lesion diagnostic rating indices and blood data one month later. Albumin and cholinesterase levels were used for the blood data. The results are shown in FIG. 8. Furthermore, in FIG 8, FIG 8(a) indicates the results for albumin, while FIG. 8(b) indicates the results for cholinesterase. In addition, data from Cases 2, 13, 16 and 17 in Tables 1 and 2 was used to carry out evaluations for albumin, while data from Case 2 was used to carry out evaluations for cholinesterase.

As shown in FIGS. 8(a) and 8(b), values for both albumin and cholinesterase decreased one month after calculation of lesion diagnostic rating indices in all cases tested. In the case of albumin, values decreased considerably in Cases 2, 13 and 17. In addition, in contrast to albumin levels being in the normal range at the time of calculation of lesion diagnostic rating indices in Cases 2 and 13, albumin levels reached the abnormal range one month after calculation of lesion diagnostic rating indices. Moreover, in contrast to the cholinesterase level at the time of calculation of the lesion diagnostic rating index in Case 2 being within the normal range at the time of calculation of the lesion diagnostic rating index, the cholinesterase level reached the abnormal range one month after calculation of the lesion diagnostic rating index.

On the basis of the above results, the lesion diagnostic rating index was determined to already exhibit values that were able to be distinguished from patients of the normal liver group (values in the vicinity of 80%) at even an earlier stage than detection of abnormalities in blood data. In addition, among patients exhibiting lesion diagnostic rating indices in the vicinity of 80%, blood data was determined to subsequently reach the abnormal range despite blood data having been in the normal range at the time of calculation of the lesion diagnostic rating indices.

On the basis of these findings, the lesion diagnostic rating index was determined to be an indicator that allows sensitive detection and diagnosis of tissue lesions or presages thereof at even an earlier stage than the appearance of abnormal values in blood tests; namely at an early stage at which blood tests are diagnosed as being 'normal'. In other words, it was determined that threshold values for assessing tissue lesions or presages thereof can be set on the basis of the lesion diagnostic rating index and other testing information, and that the threshold values alone can be used to detect tissue lesions or presages thereof. Thus, according to the lesion diagnostic rating index, a tissue lesion or presage thereof can be said to be able to be detected at an extremely early stage as compared with conventional tissue lesion diagnosis methods. As a result, treatment and diagnosis for tissue lesions can be performed at an extremely early stage.

(Example 3)

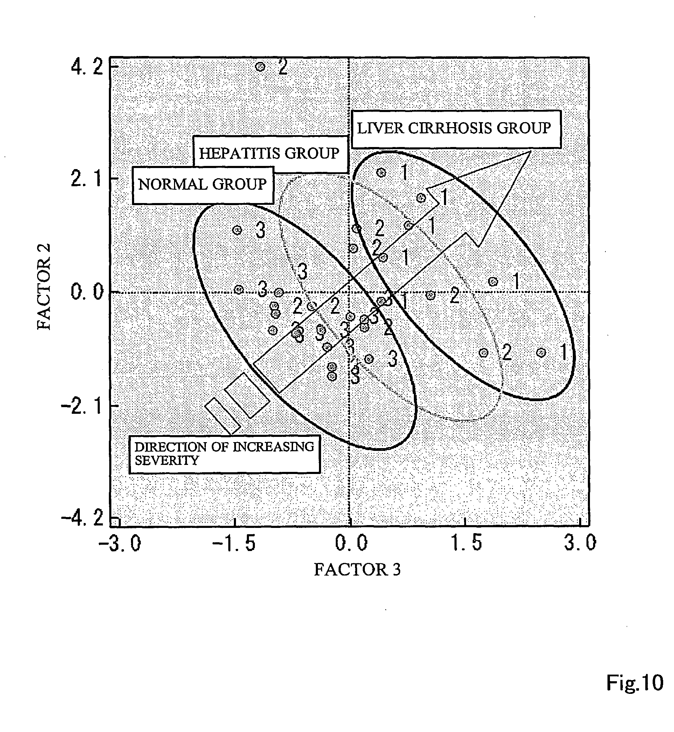

In the present example, when a total of 30 definitively diagnosed patients (consisting of liver cirrhosis patients, hepatitis patients and healthy (normal) subjects) were classified into a liver cirrhosis group, hepatitis group and normal group using data from blood test results for AST (aspartate aminotransferase, GOT), ALT (GPT), LDH (lactate dehydrogenase), Che (cholinesterase), Alb (albumin), Bil (bilirubin), $\gamma$-GPT, ALP (alkaline phosphatase) and Plt (platelets), the groups were able to be classified according to factor 2 and factor 3 indicated by the following equations. The results are shown in FIG. 10.

(Equation 3)

$$Factor\ 2 = 0.663*AST + 1.086*ALT - 0.015*LDH - 0.045*Che$$
$$-\ \ 0.218*Alb + 0.072*Bil + 0.601*\gamma\text{-}GTP + 0.582*ALP - 0.383*Plt$$

$$Factor\ 3 = 0.419*AST + 0.091*ALT - 0.052*LDH - 0.858*Che$$
$$-\ 0.891*Alb + 0.737*Bil + 0.111*\gamma\text{-}GTP - 0.135*ALP - 0.718*Plt$$

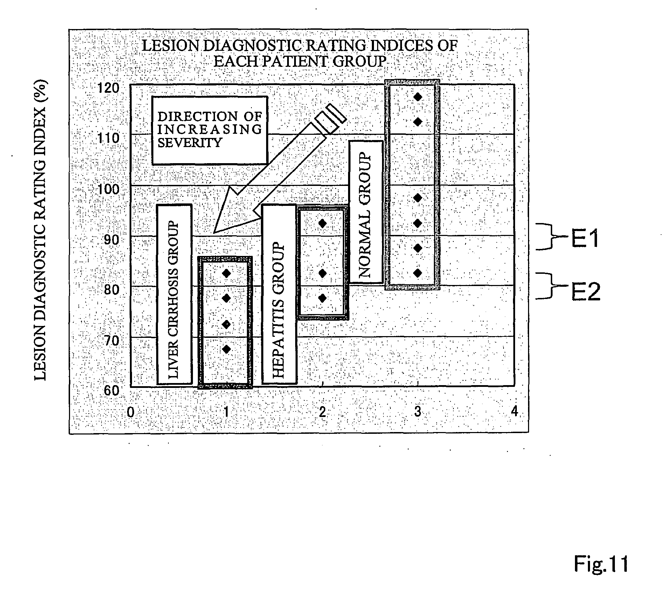

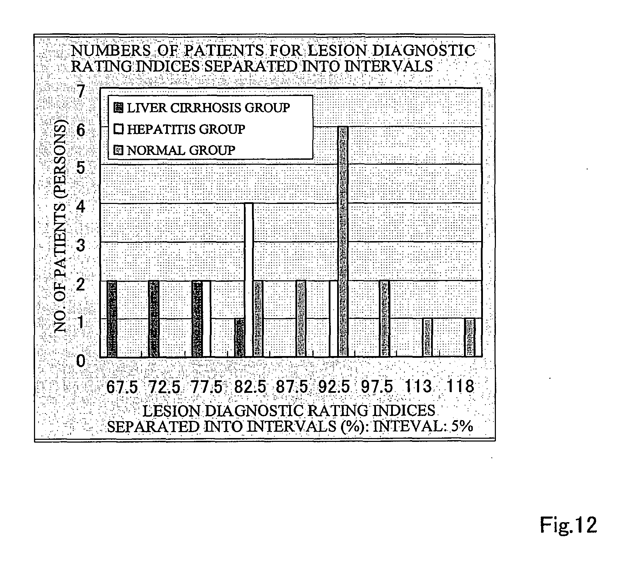

Next, the lesion diagnostic rating indices were calculated based on the equation (1) for each of these groups, and after grouping the acquired lesion diagnostic rating indices at 5% intervals, the distributions of the lesion diagnostic rating indices of the patients included in each group were investigated. The results are shown in FIG. 11. In addition, the numbers of patients in each group included in the range of each lesion diagnostic rating index were investigated. Those results are shown in FIG. 12.

As shown in FIGS. 10 to 12, the patients were able to be classified into a liver cirrhosis group, a hepatitis group and a normal group on the basis of the lesion diagnostic rating indices as well. Namely, if a threshold value is set for the lesion diagnostic rating indices in the vicinity of 90%, when the lesion diagnostic rating index is equal to or greater than the threshold value, the possibility of hepatitis or liver cirrhosis based on the presence of a tissue lesion thereof can be diagnosed as being low, or the patient can be diagnosed as normal, and when the lesion diagnostic rating index is equal to or less than the threshold value, the possibility of hepatitis or liver cirrhosis based on the presence of a tissue lesion thereof can be diagnosed as being high. In addition, if a value in the vicinity of 80% is used for the threshold value, when the lesion diagnostic rating index is equal to or less than the threshold value, the possibility of liver cirrhosis can be diagnosed as being high, and when the lesion diagnostic rating index is equal to or greater than the threshold value but equal to or less than 90%, then the possibility of hepatitis, but not liver cirrhosis, can be diagnosed as being high.

(Example 4)

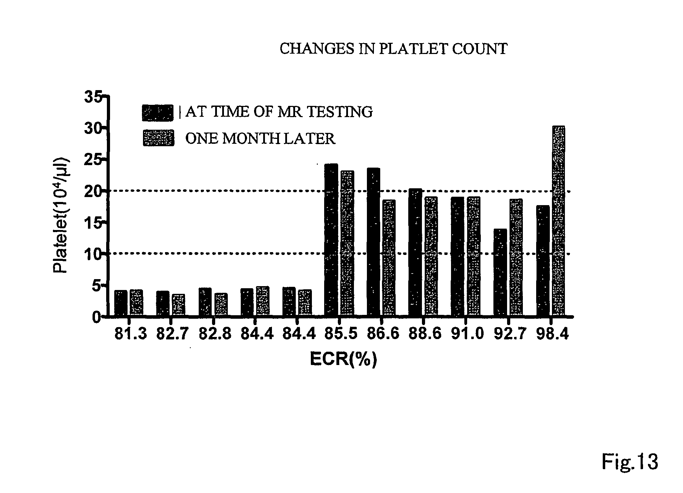

In the present example, in addition to acquiring lesion diagnostic rating indices represented by the equation (1) along with platelet test results at about the same time from 11 subjects suspected of liver disease, platelet test results were acquired from the same subjects one month later. These results are shown in FIG. 13. Furthermore, a value of 100,000 to 400,000/$\mu$l was used for the platelet reference value (normal range).

As shown in FIG. 13, when the lesion diagnostic rating index was equal to or less than 84.4%, there was no improvement in platelet test results observed at the time of initial testing or one month later. In addition, when the lesion diagnostic rating index was within the range of 85.5% to 91.0%, platelet test results after one month were equal to or had decreased as compared initial testing results. Moreover, when the lesion diagnostic rating index was equal to or greater than 92.7%, an improving trend was observed in platelet test results. Namely, it was determined that even if platelet test results are in the normal range, in the case the lesion diagnostic rating index is equal or less than a fixed value, a tendency for platelet count to decrease in the future can be detected, namely at a preliminary stage of liver disease or an extremely early stage of liver disease.

On the basis of the above, a high possibility of liver disease can be diagnosed in the case, for example, the lesion diagnostic rating index is equal to or less than about 85%, and a high possibility of the presence of a tissue change of a preliminary stage of liver disease or a tissue lesion at an extremely early stage of liver disease can be diagnosed in the case the tissue diagnostic rating index is within the range of about 85% to about 90%. Namely, the possibility of a preliminary stage of liver disease or extremely early stage of liver disease can be diagnosed as being high. In addition, when the tissue diagnostic rating index is equal to or greater than about 90%, this type of tissue change or tissue lesion can be diagnosed as being absent. Namely, the possibility of the absence of liver disease can be diagnosed as being high.

Since the present teaching is as has been previously explained, it can clearly be carried out in the following forms.

According to the present teaching, a magnetic resonance imaging device can be provided that is provided with a transmission/reception module that receives a magnetic resonance signal from a subject after having transmitted a high-frequency signal, a control module that controls the transmission/reception module such that scans are executed based on pulse sequences corresponding to both a first imaging condition unaccompanied by application of an MT pulse and a second imaging condition accompanied by the application of the MT pulse, a calculation module that calculates a lesion diagnostic rating index represented by the above-mentioned equation (1) using a signal intensity Mo of a first magnetic resonance signal received from the subject according to the first imaging condition and a signal intensity Ms of a second magnetic resonance signal received from the subject according to the second imaging condition, and a detection module that detects a lesion in living tissue or a presage thereof in the imaged region based on the lesion diagnostic rating index; wherein, when the detection module is a module that detects the lesion in living tissue or the presage thereof based on threshold information of the lesion diagnostic rating index correlated with testing information relating to a patient for which a definitive diagnosis has been made by a method differing from the diagnosis using the lesion diagnostic rating index, the threshold information may be set based on the lesion diagnostic rating index equivalent to a boundary value of the testing information relating to the patient. In this form, the threshold information can be a value equal to or greater than the lesion diagnostic rating index equivalent to the boundary value of the testing information relating to the patient.

In addition, the testing information relating to the patient may be one type or two or more types of data selected from the group consisting of testing information relating to blood in the form albumin, bilirubin and cholinesterase. Alternatively, the testing information relating to the patient may be albumin and/or cholinesterase.

In addition, the detection module can also be a module that detects the lesion in living tissue or the presage thereof at an earlier stage than detection of the lesion or the presage thereof by testing information relating to blood.

In the above-mentioned form, a display module may also be provided so that it may image and display tissue diagnostic rating indices calculated with the calculation module. In this form, the display module may carry out imaging based on threshold information of the lesion diagnostic rating indices.

The detection module may also be a module for detecting a presage of a liver disease. In this form, the liver disease may be one type or two or more types selected from the group consisting of hepatitis, liver cirrhosis and hepatocellular carcinoma.

According to the present teaching, a diagnosis method for lesion in living tissues is provided that is comprised of a detection step of detecting a lesion in living tissue of presage thereof based on the lesion diagnostic rating index represented by the above-mentioned equation (1) using a signal intensity Mo of a first magnetic resonance signal received from a subject according to a first imaging condition unaccompanied by application of an MT pulse and a signal intensity Ms of a second magnetic resonance signal received from the subject according to a second imaging condition accompanied by the application of the MT pulse, wherein the detection step is a step is carried out earlier than detection of the lesion in living tissue by testing information relating to blood.

According to the present teaching, it is clear that the present teaching can be carried out in the form of an operating method and diagnostic imaging system of a magnetic resonance imaging apparatus for carrying out the diagnosis method described above. In addition, the present teaching can also be carried out in the form of a detection method for lesion in living tissues that carries out a detection step of detecting a lesion in living tissue or presage thereof in the diagnosis method described above, a detection apparatus of a lesion in living tissue that executes this detection step, and a detection apparatus provided with an imaging module for imaging the degree of a detected lesion.

**Claims**

1.  A magnetic resonance imaging apparatus comprising:

    a transmission/reception module configured to receive a magnetic resonance signal from a subject after transmitting a high-frequency signal;

a control module configured to control the transmission/reception module such that scans are executed based on pulse sequences corresponding to a first imaging condition without application of an MT pulse and to a second imaging condition accompanied by the application of the MT pulse;

a calculation module configured to calculate a lesion diagnostic rating index represented as a function of a signal intensity Mo of a first magnetic resonance signal received from the subject according to the first imaging condition and a signal intensity Ms of a second magnetic resonance signal received from the subject according to the second imaging condition; and

a detection module configured to detect a lesion in living tissue or a presage thereof in the imaged region based on the lesion diagnostic rating index.

2. The apparatus according to claim 1, wherein the detection module is a module configured to detect the lesion in the living tissue or the presage thereof based on time-based changes in the lesion diagnostic rating index.

3. The apparatus according to claim 1 or 2, wherein the detection module is a module configured to detect the lesion in the living tissue or the presage thereof based on threshold information on the lesion diagnostic rating index that has been correlated with testing information relating to a patient for which a definitive diagnosis has been made according to information other than the lesion diagnostic rating index.

4. The apparatus according to claim 3, wherein the detection module is a module configured to perform detection based on the threshold information on the lesion diagnostic rating index that has been correlated with testing information relating to blood of the patient.

5. The apparatus according to claim 3 or 4, wherein the detection module is a module configured to detect the lesion in the living tissue or the presage thereof based on the threshold information.

6. The apparatus according to claim 5, wherein the detection module is a module configured to detect normalcy of a tissue when a value of the tissue diagnostic rating index exceeds a prescribed threshold information E1, detect a presage of a tissue lesion when the value of the tissue diagnostic rating index has exceeded a prescribed threshold information E2 but is equal to or less than the prescribed threshold information E1, and detect a presence of the tissue lesion when the value of the tissue diagnostic rating index is equal to or less than the prescribed threshold information E2.

7. The apparatus according to any of claims 3 to 6, wherein the living tissue is liver tissue, and the testing information is hematological information on one type or two or more types of components selected from a group consisting of glutamic oxaloacetic transaminase, glutamic pyruvic transaminase, albumin, bilirubin, cholinesterase and indocyanine green.

8. The apparatus according to any of claims 1 to 7, wherein the detection module is a module configured to perform detection based on:

a use of a correlation between testing information other than tissue diagnostic rating index obtained from a healthy subject group and a patient group, for which a definitive diagnosis has been made according to a method differing from diagnosis using the tissue diagnostic rating index and the lesion diagnostic rating index that has been correlated with this testing information, and

a lesion diagnostic rating index of a subject and the subject's testing information other than the lesion diagnostic rating index.

9. The apparatus according to any of claims 1 to 8, wherein the MT pulse is that in which an irradiation frequency is near a resonance frequency of water.

10. An operating method for a magnetic resonance imaging apparatus,
the method comprising:

a calculation step of calculating a lesion diagnostic rating index represented as a function of a first magnetic resonance signal Mo received from a subject according to a first imaging condition unaccompanied by application of an MT pulse and a second magnetic resonance signal Ms received from the subject according to a second imaging condition accompanied by application of the MT pulse; and

a detection step of detecting a presage of a lesion in living tissue in an imaged region based on the lesion diagnostic rating index.

11. A diagnostic imaging system, comprising:

a transmission/reception module configured to receive a magnetic resonance signal from a subject after transmitting a high-frequency signal;
a control module configured to control the transmission/reception module such that scans are executed based on pulse sequences corresponding to a first imaging condition unaccompanied by application of an MT pulse and to a second imaging condition accompanied by application of the MT pulse;
a calculation module configured to calculate a lesion diagnostic rating index represented as a function of a signal intensity Mo of a first magnetic resonance signal received from the subject according to the first imaging condition and a signal intensity Ms of a second magnetic resonance signal received from the subject according to the second imaging condition; and
a detection module configured to detect a presage of a lesion in living tissue in the imaged region based on the lesion diagnostic rating index.

12. A method of detecting a lesion in living tissue,
the method comprising:

a detection step of detecting a lesion in living tissue or a presage thereof based on a lesion diagnostic rating index represented as a function of a signal intensity Mo of a first magnetic resonance signal received from a subject according to a first imaging condition unaccompanied by application of an MT pulse and a signal intensity Ms of a second magnetic resonance signal received from the subject according to a second imaging condition accompanied by application of the MT pulse.

13. A tissue lesion detection apparatus, comprising:

a module configured to acquire a lesion diagnostic rating index represented as a function of a signal intensity Mo of a first magnetic resonance signal received from an imaged region of a subject according to a first imaging condition unaccompanied by application of an MT pulse and a signal intensity Ms of a second magnetic resonance signal received from the imaged region of the subject according to a second imaging condition accompanied by application of the MT pulse; and
a detection module configured to detect a lesion in living tissue or a presage thereof in the imaged region based on the lesion diagnostic rating index.

14. The apparatus according to claim 13, further comprising:

an imaging module configured to image a degree of the lesion in the imaged region based on the lesion diagnostic rating index in the imaged region; and
an output module configured to output an image of the degree of the lesion in the imaged region.

(a)

IMAGING PULSE

→ t

(b)

| MT PULSE | IMAGING PULSE |

→ t

Fig.1

TESTING INFORMATION RELATING TO BLOOD

Fig.2

| LESION DIAGNOSTIC RATING INDEX THRESHOLD INFORMATION | CELL STATUS |
|---|---|
| LESION DIAGNOSTIC RATING INDEX > E1 | NORMAL |
| E2 < LESION DIAGNOSTIC RATING INDEX $\leqq$ E1 | LESION WARNING SIGN PRESENT |
| LESION DIAGNOSTIC RATING INDEX $\leqq$ E2 | LESION PRESENT |

Fig.3

Fig.4

Fig.5

```
┌─────────────────────────────────────┐
│  LESION DIAGNOSTIC RATING INDEX      │
│   INDICATOR DIAGNOSIS ROUTINE        │
└─────────────────────────────────────┘
                  │
┌─────────────────────────────────────┐
│  SCAN EXECUTION ACCORDING TO         │ ～ S100
│     FIRST IMAGING CONDITION          │
└─────────────────────────────────────┘
                  │
┌─────────────────────────────────────┐
│    RECONSTRUCTION OF MR SIGNAL       │ ～ S110
└─────────────────────────────────────┘
                  │
┌─────────────────────────────────────┐
│  SCAN EXECUTION ACCORDING TO         │ ～ S120
│    SECOND IMAGING CONDITION          │
└─────────────────────────────────────┘
                  │
┌─────────────────────────────────────┐
│    RECONSTRUCTION OF MR SIGNAL       │ ～ S130
└─────────────────────────────────────┘
                  │
┌─────────────────────────────────────┐
│     CALCULATION OF LESION            │ ～ S140
│     DIAGNOSTIC RATING INDEX          │
└─────────────────────────────────────┘
                  │
┌─────────────────────────────────────┐
│         DETECTION STEP               │ ～ S150
└─────────────────────────────────────┘
                  │
┌─────────────────────────────────────┐
│              END                     │
└─────────────────────────────────────┘
```

Fig.6

Fig.7

(a) ALBUMIN

(b) CHOLINESTERASE

Fig.8

RELATIONSHIP BETWEEN CELL DENSITY AND LESION DIAGNOSTIC RATING INDEX

Fig.9

Fig.10

Fig.11

Fig.12

CHANGES IN PLATLET COUNT

Fig.13

## INTERNATIONAL SEARCH REPORT

| International application No. |
| --- |
| PCT/JP2008/067344 |

A. CLASSIFICATION OF SUBJECT MATTER
*A61B5/055*(2006.01)i

According to International Patent Classification (IPC) or to both national classification and IPC

B. FIELDS SEARCHED

Minimum documentation searched (classification system followed by classification symbols)
A61B5/055

Documentation searched other than minimum documentation to the extent that such documents are included in the fields searched

| | | | |
| --- | --- | --- | --- |
| Jitsuyo Shinan Koho | 1922-1996 | Jitsuyo Shinan Toroku Koho | 1996-2008 |
| Kokai Jitsuyo Shinan Koho | 1971-2008 | Toroku Jitsuyo Shinan Koho | 1994-2008 |

Electronic data base consulted during the international search (name of data base and, where practicable, search terms used)
JSTPlus(JDreamII), JMEDPlus(JDreamII), JST7580(JDreamII),
Wiley Inter Science

C. DOCUMENTS CONSIDERED TO BE RELEVANT

| Category* | Citation of document, with indication, where appropriate, of the relevant passages | Relevant to claim No. |
| --- | --- | --- |
| X<br>A | Shigeru MATSUSHIMA, Hideyuki NISHIOFUKU, Fumio SASAKI, Hidekazu YAMAURA, Seiichi ERA, Yasuomi KINOSADA, 'Equivalent Cross-Relaxation Rate o Mochiita Cellular Axillary Lymph Node Imaging', Dai 66 Kai Japan Radiological Society Gakujutsu Shukai Shorokushu, 28 February, 2007 (28.02. 07), page S171 | 1,3,5,8,9,13<br>2,4,6,7,10,<br>11,14 |
| X<br>A | Shigeru Matsushima, et al., "Equivalent Cross-Relaxation Rate Imaging for Sentinel Lymph Node Biopsy in Breast Carcinoma", Magnetic Resonance in Medicine, 2005.11, Vol.54, p1300-p1304 | 1,3,5,8,9,13<br>2,4,6,7,10,<br>11,14 |

| | | | |
| --- | --- | --- | --- |
| ☒ | Further documents are listed in the continuation of Box C. | ☐ | See patent family annex. |

| | | |
| --- | --- | --- |
| * | Special categories of cited documents: | |
| "A" | document defining the general state of the art which is not considered   to be of particular relevance | "T" later document published after the international filing date or priority date and not in conflict with the application but cited to understand the principle or theory underlying the invention |
| "E" | earlier application or patent but published on or after the international filing date | "X" document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" document member of the same patent family |

| Date of the actual completion of the international search | Date of mailing of the international search report |
| --- | --- |
| 09 October, 2008 (09.10.08) | 21 October, 2008 (21.10.08) |

| Name and mailing address of the ISA/<br>Japanese Patent Office | Authorized officer |
| --- | --- |
| Facsimile No. | Telephone No. |

Form PCT/ISA/210 (second sheet) (April 2007)

**INTERNATIONAL SEARCH REPORT**

International application No.

PCT/JP2008/067344

C (Continuation). DOCUMENTS CONSIDERED TO BE RELEVANT

| Category* | Citation of document, with indication, where appropriate, of the relevant passages | Relevant to claim No. |
|---|---|---|
| X<br>A | Haruo HANYU, Yukari IMON, Masaru TAKASAKI,<br>Hiroaki SHINDO, Kimihiko ABE, 'No Kosoku ni<br>Okeru magnetization transfer ratio no Henka',<br>Japanese Journal of Stroke, 25 October, 1997<br>(25.10.97), Vol.19, No.5, pages 361 to 365 | 1,9,13<br>2-8,10,11,14 |

Form PCT/ISA/210 (continuation of second sheet) (April 2007)

## INTERNATIONAL SEARCH REPORT

| International application No. |
|---|
| PCT/JP2008/067344 |

**Box No. II      Observations where certain claims were found unsearchable (Continuation of item 2 of first sheet)**

This international search report has not been established in respect of certain claims under Article 17(2)(a) for the following reasons:

1. ☒ Claims Nos.: 12
   because they relate to subject matter not required to be searched by this Authority, namely:
   Claim 12 relates to a method for detecting a lesion of a living tissue, in which the disease state is determined.  Therefore, claim 12 pertains to a diagnosis method, and thus relates to a subject matter which this International Searching Authority    (Continues to extra sheet.)

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box No. III      Observations where unity of invention is lacking (Continuation of item 3 of first sheet)**

This International Searching Authority found multiple inventions in this international application, as follows:
   The matter common to the inventions of claims 1-14 is considered to be equivalent to the matter stated in claim 1.
   However, the search has revealed that the matter stated in claim 1 is not novel since it is disclosed in document: Shigeru MATSUSHIMA, Hideyuki NISHIOFUKU, Fumio SASAKI, Hidekazu YAMAURA, Seiichi ERA, Yasuomi KINOSADA, 'Equivalent Cross-Relaxation Rate o Mochiita Cellular Axillary Lymph Node Imaging', Dai 66 Kai Japan Radiological Society Gakujutsu Shukai Shorokushu, 28 February, 2007 (28.02.07), page S171.
   (continued to extra sheet)

1. ☐ As all required additional search fees were timely paid by the applicant, this international search report covers all searchable claims.

2. ☒ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant.   Consequently, this international search report is restricted to the invention first mentioned in the claims; it is covered by claims Nos.:

**Remark on Protest**
the

☐ The additional search fees were accompanied by the applicant's protest and, where applicable, payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (April 2007)

| INTERNATIONAL SEARCH REPORT | International application No. |
|---|---|
| | PCT/JP2008/067344 |

Continuation of Box No.II-1 of continuation of first sheet(2)

is not required, under the provisions of PCT Article 17(2)(a)(i) and PCT Rule 39.1(iv), to search.

Continuation of Box No.III of continuation of first sheet(2)

In consequence, since the matter stated in claim 1 makes no contribution over the prior art, this matter cannot be a special technical feature within the meaning of PCT Rule 13.2, second sentence.

Therefore, the inventions of claims 1-14 obviously do not satisfy the requirement of unity of invention.

Form PCT/ISA/210 (extra sheet) (April 2007)

**EP 2 208 462 A1**

**Patent documents cited in the description**

- JP H11313810 B

- JP 2006116299 A

**Non-patent literature cited in the description**

- *Magnetic Resonance in Medicine,* 2005, vol. 54, 1300-1304